# EUROPEAN PATENT APPLICATION

(11) **EP 4 193 951 A1**
(43) Date of publication of application: **14.06.2023**
(21) Application number: 22212771.4
(22) Date of filing: 12.12.2022
(51) Int. Cl.: A61B 34/00

(54) **FOOT PEDAL TWO STAGE BUTTON AND REARRANGE FOR A SURGICAL ROBOTIC SYSTEM**

(30) Priority: 13.12.2021 US 202163288675 P; 14.11.2022 US 202217986047
(71) Applicant: Covidien LP, Mansfield, MA 02048 (US)
(72) Inventor: JOSEPH, Daniel A., Boulder, 80301 (US)
(74) Representative: Maschio, Antonio

(57) **Abstract**

Devices to control a movement or instrument function of a robotic arm of a surgical robotic system include a foot pedal configured to generate a first input signal when the foot pedal is moved to a first activation position and a second input signal, different from the first input signal, when the foot pedal is moved to a second activation position. The first activation position may be associated with a first specific movement or instrument function of a robotic arm. The second activation position may be associated with a second specific movement or instrument function of the robotic arm. The foot pedal is configured to send the first input signal or the second input signal to a surgical console to be utilized to remotely control the respective specific movement or instrument function of the robotic arm.

## Description

### CROSS-REFERENCE TO RELATED APPLICATIONS

This application claims the benefit of the filing date of provisional U.S. Patent Application No. 63/288,675 filed on December 13, 2021.

### BACKGROUND

Surgical robotic systems may include a surgical console controlling one or more surgical robotic arms, each having a surgical instrument having an end effector (e.g., forceps or grasping instrument). In operation, a user provides input to the surgical robotic systems through one or more interface devices, which are interpreted by a control tower of a surgical console as movement commands for moving the surgical robotic arm. Based on the user inputs, the surgical console sends movement commands to the robotic arm so that the robotic arm is moved to a position over a patient and the surgical instrument is guided into a small incision via a surgical access port or a natural orifice of a patient to position the end effector at a work site within the patient's body.

### SUMMARY

This disclosure describes devices, systems, and methods to control a movement or instrument function of a robotic arm of a surgical robotic system.

One embodiment of the present disclosure is a foot pedal system for a surgical robotic system. The foot pedal system includes a foot pedal with a foot plate and a support. The foot plate includes a first portion and a second portion. The foot pedal is configured to generate a first input signal in response to the foot pedal being moved to a first activation position corresponding to at least one of a first movement command or a first instrument function. The foot pedal is configured to generate a second input signal in response to the foot pedal being moved to a second activation position corresponding to at least one of a second movement command or a second instrument function. The second input signal is different from the first input signal. The foot pedal is configured to send the first input signal or the second input signal to a surgical console configured to remotely control a surgical robotic system based on the first input signal or the second input signal.

In aspects, the foot pedal is a two-stage foot pedal.

In aspects, the two-stage foot pedal is configured to pivot to the first activation position when a force is applied to move the first portion of the foot plate to the first activation position by a foot of a user and to pivot to the second activation position when a force is applied to move the first portion of the foot plate to the second activation position by the foot of the user.

In aspects, the foot pedal is a rocker foot pedal.

In aspects, the rocker foot pedal is configured to pivot to the first activation position when a force is applied to move the first portion of the foot plate to the first activation position by a foot of the user and to pivot to the second activation position when a force is applied to the second portion of the foot plate to move the second portion of the foot plate to the second activation position by the foot of the user.

In aspects, the foot pedal is color coded to identify specific movements or instrument functions of the robotic arm assigned to the foot pedal.

In aspects, a video display of the surgical robotic system displays the foot pedal and the color code of the foot pedal.

In aspects, the foot pedal includes a location sensor to indicate a location of a foot relative to the foot pedal.

In aspects, the location sensor is one of a capacitive proximity sensor, an optical sensor with reflected light, or a video camera system.

In aspects, the foot pedal includes a light, and the light is configured to light up when a foot hovers over the foot pedal or when the foot pedal is depressed by a foot of the user to the first activation position or the second activation position.

In aspects, the instrument function includes at least one of bipolar coagulation, tissue cutting, stapling, monopolar power level, or ultrasonic power level.

Another embodiment of the present disclosure is a surgical robotic system. The system includes a robotic arm including a surgical instrument, and a surgical console including at least one foot pedal. The foot pedal is configured to generate a first input signal in response to the foot pedal being moved to a first activation position corresponding to at least one of a first movement command or a first instrument function. The foot pedal is configured to generate a second input signal in response to the foot pedal being moved to a second activation position corresponding to at least one of a second movement command or a second instrument function. The second input signal is different from the first input signal. The foot pedal is configured to send the first input signal or the second input signal to a surgical console configured to remotely control a surgical robotic system based on the first input signal or the second input signal.

Another embodiment of the present disclosure is a method for controlling a movement or instrument function of a robotic arm of a surgical robotic system. The method includes a foot pedal generating a first input signal in response to the foot pedal being moved to a first activation position corresponding to at least one of a first movement command or a first instrument function. The method includes the foot pedal generating a second input signal in response to the foot pedal being moved to a second activation position corresponding to at least one of a second movement command or a second instrument function. The second input signal is different from the first input signal. The method includes the foot pedal sending the first input signal or the second input signal to a surgical console configured to remotely control a surgical robotic system based on the first input signal or the second input signal.

### BRIEF DESCRIPTION OF THE DRAWINGS

Various embodiments of the present disclosure are described herein with reference to the drawings wherein:
FIG. 1 is a schematic illustration of a surgical robotic system including a control tower, a console, and one or more surgical robotic arms each disposed on a mobile cart according to an embodiment of the present disclosure;
FIG. 2 is a perspective view of a surgical robotic arm of the surgical robotic system of FIG. 1 according to an embodiment of the present disclosure;
FIG. 3 is a perspective view of a setup arm with the surgical robotic arm of the surgical robotic system of FIG. 1 according to an embodiment of the present disclosure;
FIG. 4 is a schematic diagram of a computer architecture of the surgical robotic system of FIG. 1 according to an embodiment of the present disclosure;
FIG.5a is a side view of a two-stage foot pedal of the surgical robotic system of FIG. 1 in a neutral position according to an embodiment of the present disclosure;
FIG.5b is a side view of a two-stage foot pedal of the surgical robotic system of FIG. 1 in a first activation position according to an embodiment of the present disclosure;
FIG.5c is a side view of a two-stage foot pedal of the surgical robotic system of FIG. 1 in a second activation position according to an embodiment of the present disclosure;
FIG.6a is a side view of a rocker foot pedal of the surgical robotic system of FIG. 1 in a neutral position according to an embodiment of the present disclosure;
FIG.6b is a side view of a rocker foot pedal of the surgical robotic system of FIG. 1 in a first activation position according to an embodiment of the present disclosure;
FIG.6c is a side view of a rocker foot pedal of the surgical robotic system of FIG. 1 in a second activation position according to an embodiment of the present;
FIG.7a is a rear view of a side rocker foot pedal of the surgical robotic system of FIG. 1 in a neutral position according to an embodiment of the present disclosure;
FIG.7b is a rear view of a side rocker foot pedal of the surgical robotic system of FIG. 1 in a first activation position according to an embodiment of the present disclosure;
FIG.7c is a rear view of a side rocker foot pedal of the surgical robotic system of FIG. 1 in a second activation position according to an embodiment of the present;
FIG.8a is a side view of a two-stage foot pedal of the surgical robotic system of FIG. 1 in a neutral position according to an embodiment of the present disclosure;
FIG.8b is a side view of a two-stage foot pedal of the surgical robotic system of FIG. 1 in a first activation position according to an embodiment of the present disclosure;
FIG.8c is a side view of a two-stage foot pedal of the surgical robotic system of FIG. 1 in a second activation position according to an embodiment of the present disclosure;
FIG.9a is a side view of a variable output foot pedal of the surgical robotic system of FIG. 1 in a neutral position according to an embodiment of the present disclosure;
FIG.9b is a side view of a variable output foot pedal of the surgical robotic system of FIG. 1 in a first activation position according to an embodiment of the present disclosure;
FIG.9c is a side view of a variable output foot pedal of the surgical robotic system of FIG. 1 in a second activation position according to an embodiment of the present disclosure;
FIG.10 is a side view of a roller ball foot pedal of the surgical robotic system of FIG. 1 according to an embodiment of the present disclosure; and
FIG.11 is a flow diagram of a method for controlling a movement or instrument function of a robotic arm of a surgical robotic system according to an embodiment of the present disclosure.

### DETAILED DESCRIPTION

Embodiments of the presently disclosed surgical robotic system are described in detail with reference to the drawings, in which like reference numerals designate identical or corresponding elements in each of the several views.

As will be described in detail below, the present disclosure is directed to a surgical robotic system, which includes a surgical console, a control tower, and one or more mobile carts having a surgical robotic arm coupled to a setup arm. The surgical console receives user input through one or more interface devices, which are interpreted by the control tower as movement commands for moving the surgical robotic arm. The surgical robotic arm includes a controller, which is configured to process the movement command and to generate a torque command for activating one or more actuators of the robotic arm, which would, in turn, move the robotic arm in response to the movement command.

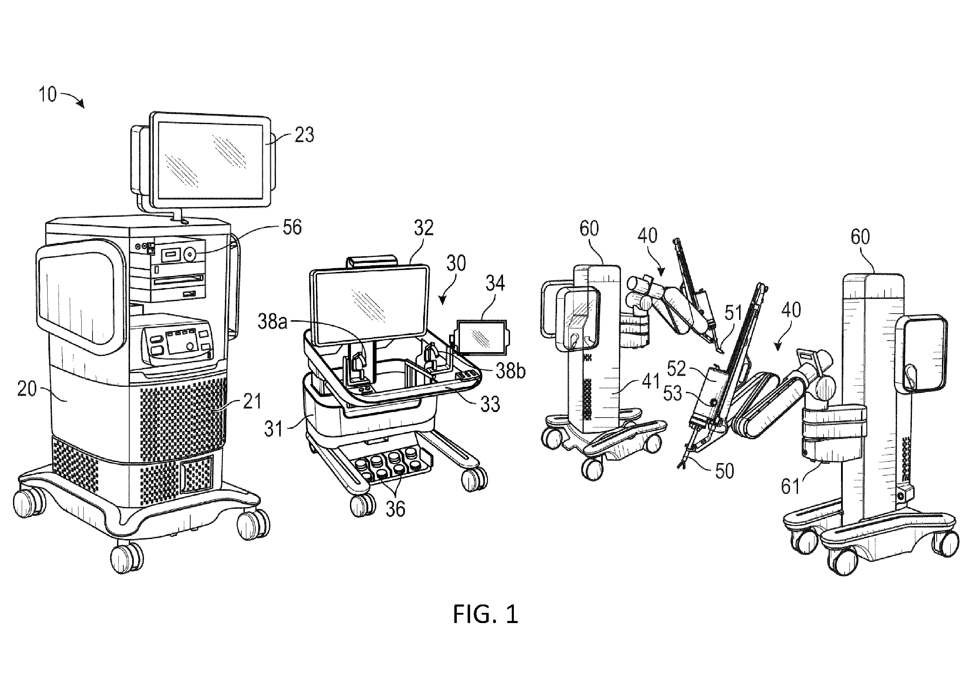

With reference to FIG. 1, a surgical robotic system 10 includes a control tower 20, which is connected to all of the components of the surgical robotic system 10 including a surgeon console 30 and one or more mobile carts 60. Each of the mobile carts 60 includes a robotic arm 40 having a surgical instrument 50 removably coupled thereto. The robotic arms 40 is also coupled to the mobile cart 60. The system 10 may include any number of mobile carts 60 and/or robotic arms 40.

The surgical instrument 50 is configured for use during minimally invasive surgical procedures. In embodiments, the surgical instrument 50 may be configured for open surgical procedures. In embodiments, the surgical instrument 50 may be an endoscope, such as an endoscopic camera 51, configured to provide a video feed for the user. In further embodiments, the surgical instrument 50 may be an electrosurgical forceps configured to seal tissue by compressing tissue between jaw members and applying electrosurgical current thereto. In yet further embodiments, the surgical instrument 50 may be a surgical stapler including a pair of jaws configured to grasp and clamp tissue while deploying a plurality of tissue fasteners, e.g., staples, and cutting stapled tissue.

One of the robotic arms 40 may include the endoscopic camera 51 configured to capture video of the surgical site. The endoscopic camera 51 may be a stereoscopic endoscope configured to capture two side-by-side (i.e., left and right) images of the surgical site to produce a video stream of the surgical scene. The endoscopic camera 51 is coupled to a video processing device 56, which may be disposed within the control tower 20. The video processing device 56 may be any computing device as described below configured to receive the video feed from the endoscopic camera 51, perform the image processing based on the depth estimating algorithms of the present disclosure, and output the processed video stream.

The surgeon console 30 includes a first display 32, which displays a video feed of the surgical site provided by camera 51 of the surgical instrument 50 disposed on the robotic arm 40, and a second display 34, which displays a user interface for controlling the surgical robotic system 10. The first and second displays 32 and 34 are touchscreens allowing for displaying various graphical user inputs.

The surgeon console 30 also includes a plurality of user interface devices, such as foot pedals 36 and a pair of handle controllers 38a and 38b which are used by a user to remotely control robotic arms 40. The surgeon console further includes an armrest 33 used to support clinician's arms while operating the handle controllers 38a and 38b.

The control tower 20 includes a display 23, which may be a touchscreen, and outputs on the graphical user interfaces (GUIs). The control tower 20 also acts as an interface between the surgeon console 30 and one or more robotic arms 40. In particular, the control tower 20 is configured to control the robotic arms 40, such as to move the robotic arms 40 and the corresponding surgical instrument 50, based on a set of programmable instructions and/or input commands from the surgeon console 30, in such a way that robotic arms 40 and the surgical instruments 50 execute a desired movement sequence in response to input from the foot pedals 36 and the handle controllers 38a and 38b.

Each of the control tower 20, the surgeon console 30, and the robotic arm 40 includes a respective computer 21, 31, 41. The computers 21, 31, 41 are interconnected to each other using any suitable communication network based on wired or wireless communication protocols. The term "network," whether plural or singular, as used herein, denotes a data network, including, but not limited to, the Internet, Intranet, a wide area network, or a local area network, and without limitation as to the full scope of the definition of communication networks as encompassed by the present disclosure. Suitable protocols include, but are not limited to, transmission control protocol/internet protocol (TCP/IP), datagram protocol/internet protocol (UDP/IP), and/or datagram congestion control protocol (DCCP). Wireless communication may be achieved via one or more wireless configurations, e.g., radio frequency, optical, Wi-Fi, Bluetooth (an open wireless protocol for exchanging data over short distances, using short length radio waves, from fixed and mobile devices, creating personal area networks (PANs), ZigBee^{®} (a specification for a suite of high level communication protocols using small, low-power digital radios based on the IEEE 122.15.4-2003 standard for wireless personal area networks (WPANs)).

The computers 21, 31, 41 may include any suitable processor (not shown) operably connected to a memory (not shown), which may include one or more of volatile, non-volatile, magnetic, optical, or electrical media, such as read-only memory (ROM), random access memory (RAM), electrically-erasable programmable ROM (EEPROM), non-volatile RAM (NVRAM), or flash memory. The processor may be any suitable processor (e.g., control circuit) adapted to perform the operations, calculations, and/or set of instructions described in the present disclosure including, but not limited to, a hardware processor, a field programmable gate array (FPGA), a digital signal processor (DSP), a central processing unit (CPU), a microprocessor, and combinations thereof. Those skilled in the art will appreciate that the processor may be substituted for by using any logic processor (e.g., control circuit) adapted to execute algorithms, calculations, and/or set of instructions described herein.

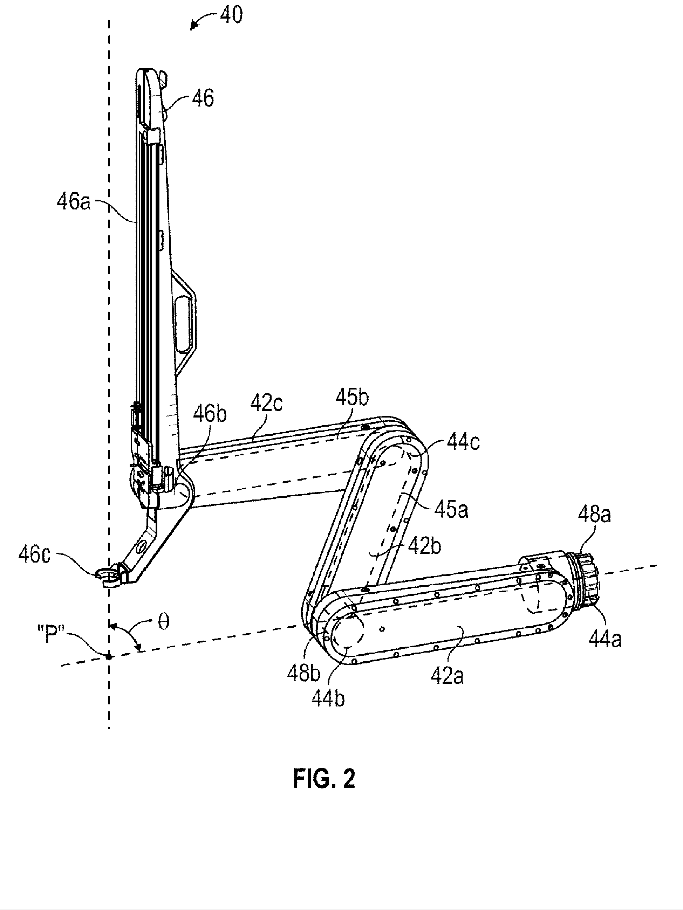

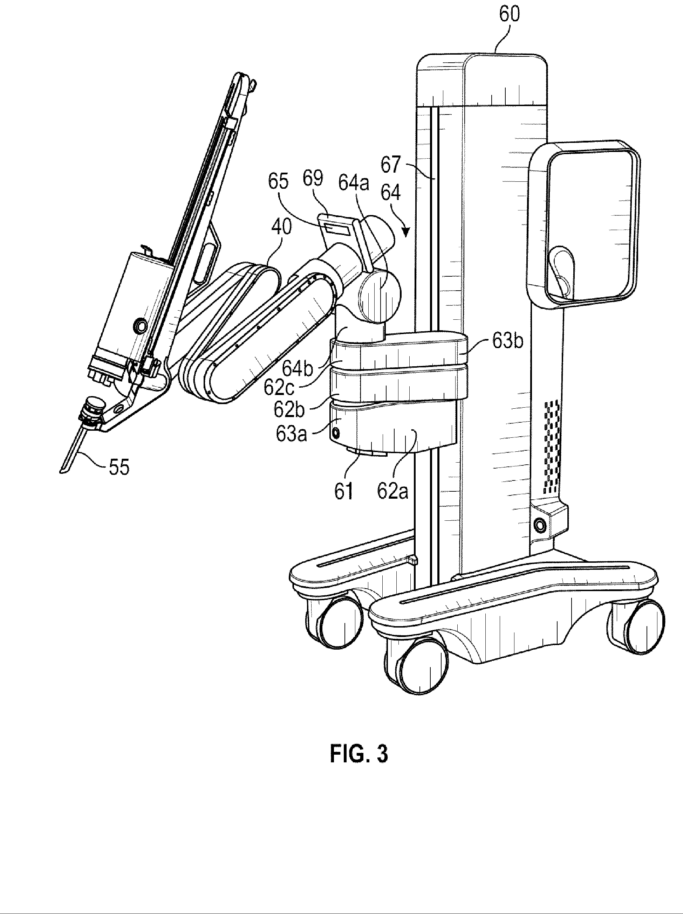

With reference to FIG. 2, each of the robotic arms 40 may include a plurality of links 42a, 42b, 42c, which are interconnected at joints 44a, 44b, 44c, respectively. Other configurations of links and joints may be utilized as known by those skilled in the art. The joint 44a is configured to secure the robotic arm 40 to the mobile cart 60 and defines a first longitudinal axis. With reference to FIG. 3, the mobile cart 60 includes a lift 67 and a setup arm 61, which provides a base for mounting of the robotic arm 40. The lift 67 allows for vertical movement of the setup arm 61. The mobile cart 60 also includes a display 69 for displaying information pertaining to the robotic arm 40. In embodiments, the robotic arm 40 may include any type and/or number of joints.

The setup arm 61 includes a first link 62a, a second link 62b, and a third link 62c, which provide for lateral maneuverability of the robotic arm 40. The links 62a, 62b, 62c are interconnected at joints 63a and 63b, each of which may include an actuator (not shown) for rotating the links 62b and 62b relative to each other and the link 62c. In particular, the links 62a, 62b, 62c are movable in their corresponding lateral planes that are parallel to each other, thereby allowing for extension of the robotic arm 40 relative to the patient (e.g., surgical table). In embodiments, the robotic arm 40 may be coupled to the surgical table (not shown). The setup arm 61 includes controls 65 for adjusting movement of the links 62a, 62b, 62c as well as the lift 67. In embodiments, the setup arm 61may include any type and/or number of joints.

The third link 62c may include a rotatable base 64 having two degrees of freedom. In particular, the rotatable base 64 includes a first actuator 64a and a second actuator 64b. The first actuator 64a is rotatable about a first stationary arm axis which is perpendicular to a plane defined by the third link 62c and the second actuator 64b is rotatable about a second stationary arm axis which is transverse to the first stationary arm axis. The first and second actuators 64a and 64b allow for full three-dimensional orientation of the robotic arm 40.

The actuator 48b of the joint 44b is coupled to the joint 44c via the belt 45a, and the joint 44c is in turn coupled to the joint 46b via the belt 45b. Joint 44c may include a transfer case coupling the belts 45a and 45b, such that the actuator 48b is configured to rotate each of the links 42b, 42c and a holder 46 relative to each other. More specifically, links 42b, 42c, and the holder 46 are passively coupled to the actuator 48b which enforces rotation about a pivot point "P" which lies at an intersection of the first axis defined by the link 42a and the second axis defined by the holder 46. In other words, the pivot point "P" is a remote center of motion (RCM) for the robotic arm 40. Thus, the actuator 48b controls the angle θ between the first and second axes allowing for orientation of the surgical instrument 50. Due to the interlinking of the links 42a, 42b, 42c, and the holder 46 via the belts 45a and 45b, the angles between the links 42a, 42b, 42c, and the holder 46 are also adjusted in order to achieve the desired angle θ. In embodiments, some or all of the joints 44a, 44b, 44c may include an actuator to obviate the need for mechanical linkages.

The joints 44a and 44b include an actuator 48a and 48b configured to drive the joints 44a, 44b, 44c relative to each other through a series of belts 45a and 45b or other mechanical linkages such as a drive rod, a cable, or a lever and the like. In particular, the actuator 48a is configured to rotate the robotic arm 40 about a longitudinal axis defined by the link 42a.

With reference to FIG. 2, the holder 46 defines a second longitudinal axis and configured to receive an instrument drive unit (IDU) 52 (FIG. 1). The IDU 52 is configured to couple to an actuation mechanism of the surgical instrument 50 and the camera 51 and is configured to move (e.g., rotate) and actuate the instrument 50 and/or the camera 51. IDU 52 transfers actuation forces from its actuators to the surgical instrument 50 to actuate components (e.g., end effector) of the surgical instrument 50. The holder 46 includes a sliding mechanism 46a, which is configured to move the IDU 52 along the second longitudinal axis defined by the holder 46. The holder 46 also includes a joint 46b, which rotates the holder 46 relative to the link 42c. During endoscopic procedures, the instrument 50 may be inserted through an endoscopic port 55 (FIG. 3) held by the holder 46. The holder 46 also includes a port latch 46c for securing the port 55 to the holder 46 (FIG. 2).

The robotic arm 40 also includes a plurality of manual override buttons 53 (FIG. 1) disposed on the IDU 52 and the setup arm 61, which may be used in a manual mode. The user may press one or more of the buttons 53 to move the component associated with the button 53.

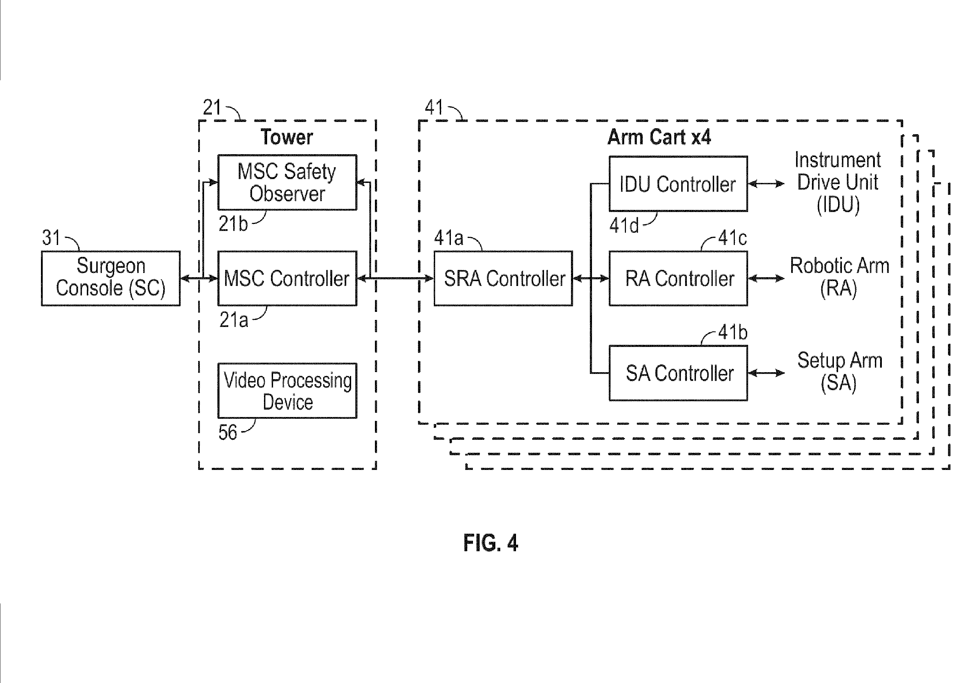

With reference to FIG. 4, each of the computers 21, 31, 41 of the surgical robotic system 10 may include a plurality of controllers, which may be embodied in hardware and/or software. The computer 21 of the control tower 20 includes a controller 21a and safety observer 21b. The controller 21a receives data from the computer 31 of the surgical console 30 about the current position and/or orientation of the handle controllers 38a and 38b and the state of the foot pedals 36 and other buttons. The controller 21a processes these input positions to determine desired drive commands for each joint of the robotic arm 40 and/or the instrument drive unit 52 and communicates these to the computer 41 of the robotic arm 40. The controller 21a also receives back the actual joint angles and uses this information to determine force feedback commands that are transmitted back to the computer 31 of the surgical console 30 to provide haptic feedback through the handle controllers 38a and 38b. The safety observer 21b performs validity checks on the data going into and out of the controller 21a and notifies a system fault handler if errors in the data transmission are detected to place the computer 21 and/or the surgical robotic system 10 into a safe state.

The computer 41 includes a plurality of controllers, namely, a main cart controller 41a, a setup arm controller 41b, a robotic arm controller 41c, and an instrument drive unit (IDU) controller 41d. The main cart controller 41a receives and processes joint commands from the controller 21a of the computer 21 and communicates them to the setup arm controller 41b, the robotic arm controller 41c, and the IDU controller 41d. The main cart controller 41a also manages instrument exchanges and the overall state of the mobile cart 60, the robotic arm 40, and the instrument drive unit 52. The main cart controller 41a also communicates actual joint angles back to the controller 21a.

The setup arm controller 41b controls each of joints 63a and 63b, and the rotatable base 64 of the setup arm 62 and calculates desired motor movement commands (e.g., motor torque) for the pitch axis and controls the brakes. The robotic arm controller 41c controls each joint 44a and 44b of the robotic arm 40 and calculates desired motor torques required for gravity compensation, friction compensation, and closed loop position control of the robotic arm 40. The robotic arm controller 41c calculates a movement command based on the calculated torque. The calculated motor commands are then communicated to one or more of the actuators 48a and 48b in the robotic arm 40. The actual joint positions are then transmitted by the actuators 48a and 48b back to the robotic arm controller 41c.

The IDU controller 41d receives desired joint angles for the surgical instrument 50, such as wrist and jaw angles, and computes desired currents for the motors in the instrument drive unit 52. The IDU controller 41d calculates actual angles based on the motor positions and transmits the actual angles back to the main cart controller 41a.

The robotic arm 40 is controlled as follows. Initially, a pose of the handle controller controlling the robotic arm 40, e.g., the handle controller 38a, is transformed into a desired pose of the robotic arm 40 through a hand eye transform function executed by the controller 21a. The hand eye function, as well as other functions described herein, is/are embodied in software executable by the controller 21a or any other suitable controller described herein. The pose of the handle controller 38a may be embodied as a coordinate position and role-pitch-yaw ("RPY") orientation relative to a coordinate reference frame, which is fixed to the surgical console 30. The desired pose of the instrument 50 is relative to a fixed frame on the robotic arm 40. The pose of the handle controller 38a is then scaled by a scaling function executed by the controller 21a. In some instances, the coordinate position may be scaled down and the orientation may be scaled up by the scaling function. In addition, the controller 21a also executes a clutching function, which disengages the handle controller 38a from the robotic arm 40. In particular, the main cart controller 21a stops transmitting movement commands from the handle controller 38a to the robotic arm 40 if certain movement limits or other thresholds are exceeded and in essence acts like a virtual clutch mechanism, e.g., limits mechanical input from effecting mechanical output.

The desired pose of the robotic arm 40 is based on the pose of the handle controller 38a and is then passed by an inverse kinematics function executed by the controller 21a. The inverse kinematics function calculates angles for the joints 44a, 44b, and 44c of the robotic arm 40 that achieve the scaled and adjusted pose input by the handle controller 38a. The calculated angles are then passed to the robotic arm controller 41c, which includes a joint axis controller having a proportional-derivative (PD) controller, the friction estimator module, the gravity compensator module, and a two-sided saturation block, which is configured to limit the commanded torque of the motors of the joints 44a, 44b, 44c.

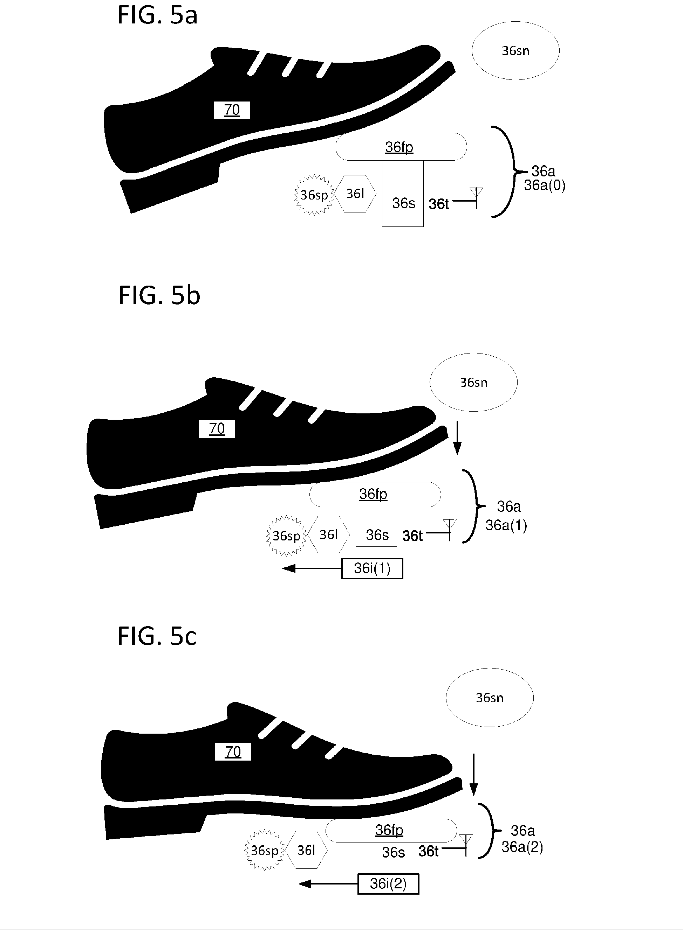

With reference to FIGS. 5a-5c through FIGS. 8a-8c, each of the foot pedals 36 of FIG. 1 may be configured to control more than one function with a two-stage button or actuation and may be rearranged to include at least a first activation position and a second activation position. In embodiments, each of the foot pedals 36 may be a multi-stage switch configured to control a plurality of functions of the system 10.

FIGS. 5a-5c are side views of various foot pedal activation positions of a two-stage foot pedal 36a. FIG.5a is a side view of two-stage foot pedal 36a in a neutral position. Two-stage foot pedal 36a may include a foot plate 36fp and a support 36s. Foot plate 36fp may be attached to and/or supported by support 36s. Foot plate 36fp may be configured to be supported by support 36s in a neutral position 36a(0) when no force is applied to foot plate 36fp by a foot 70 of a user. Two-stage foot pedal 36a may not generate an input signal when foot plate 36fp is not moved and in neutral position 36a(0).

FIG.5b is a side view of two-stage foot pedal 36a in a first activation position. Foot plate 36fp may be configured to move to a first activation position 36a(1) when a force is applied to move foot plate36fp to first activation position 36a(1) by a foot 70 of a user. Two-stage foot pedal 36a may be configured to generate tactical feedback to a user such as a physical click when foot plate 36fp is moved to first activation position 36a(1). Two-stage foot pedal 36a may also generate a noise by a speaker 36sp and/or illuminate a light 361 when foot plate 36fp is moved to first activation position 36a(1). First activation position 36a(1) may be associated with a specific movement or instrument function of robotic arms 40 of FIG. 1. Two-stage foot pedal 36a may generate a first input signal 36i(1) when foot plate 36fp is moved to first activation position 36a(1). Two-stage foot pedal 36a may send first input signal 36i(1) to surgical console 30, control tower 20 and/or at least one of the robotic arms 40 of FIG. 1 by transmitter 36t of foot pedal 36a. First input signal 36i(1), from two-stage foot pedal 36a, may be utilized by surgical console 30 to remotely control a specific movement or instrument function of robotic arms 40 of FIG. 1. Instrument function, remotely controlled by first input signal 36i(1) from two-stage foot pedal 36a, may include bipolar coagulation, tissue cutting, stapling, monopolar power level, ultrasonic power level, etc.

While a wireless transmitter 36t is illustrated, it is contemplated and within the scope of the disclosure that hardwired connections may be used to send first input signal 36i(1) to the surgical console 30, the control tower 20 and/or the robotic arm(s) 40.

FIG.5c is a side view of two-stage foot pedal 36a in a second activation position. Foot plate 36fp may be configured to move to a second activation position 36a(2) when a force is applied to move foot plate 36fp to second activation position 36a(2) by a foot 70 of a user. Two-stage foot pedal 36a may generate tactical feedback to a user such as a physical click when foot plate 36fp is moved to second activation position 36a(2). Two-stage foot pedal 36a may generate a noise by speaker 36sp and/or illuminate light 361 when foot plate 36fp is moved to second activation position 36a(2). A noise and/or illumination generated by two-stage foot pedal 36a may be different for first activation position 36a(1) than for second activation position 36a(2) so that a user can easily distinguish between the two activation positions. For example, two-stage foot pedal 36a may illuminate light 361 when foot plate 36fp is moved to first activation position 36a(1) and two-stage foot pedal 36a may generate a buzzer noise by speaker 36sp when foot plate 36fp is moved to second activation position 36a(2). Second activation position 36a(2) may be associated with a specific movement or instrument function of robotic arms 40 of FIG. 1. Two-stage foot pedal 36a may generate a second input signal 36i(2) when foot plate 36fp is moved to second activation position 36a(2). Two-stage foot pedal 36a may send second input signal 36i(2) to surgical console 30, control tower 20 and/or at least one of the robotic arms 40 of FIG. 1 by transmitter 36t of foot pedal 36a. As previously stated, hardwired connections may be used to send second input signal 36i(2) to the surgical console 30 the control tower 20 and/or the robotic arm(s) 40. Second input signal 36i(2), from two-stage foot pedal 36a, may be utilized by surgical console 30 to remotely control a specific movement or instrument function of robotic arms 40 of FIG. 1, different from the movement or function controlled by first input signal 36i(1). Instrument function remotely controlled by second input signal 36i(2) from two-stage foot pedal 36a may include bipolar coagulation, tissue cutting, stapling, monopolar power level, ultrasonic power level, etc., or may include the same instrument function as first input signal 36i(1) however at a relatively higher degree of power or intensity.

As shown in FIGS. 5a-5c, two-stage foot pedal 36a may generate first input signal 36i(1) or second input signal 36i(2) depending on movement of foot plate 36fp to first activation position 36a(1) or second activation position 36a(2) by a foot 70 of a user. The two activation positions 36a(1) and 36a(2) of two-stage foot pedal 36a may allow a user to control two different functions or instrument settings of surgical robotic system 10 with two-stage foot pedal 36a. In embodiments, as mentioned above, first input signal 36i(1) may be for a first power setting for an instrument of surgical robotic system 10 and second input signal 36i(2) may be for a second, higher power setting for the same instrument of surgical robotic system 10.

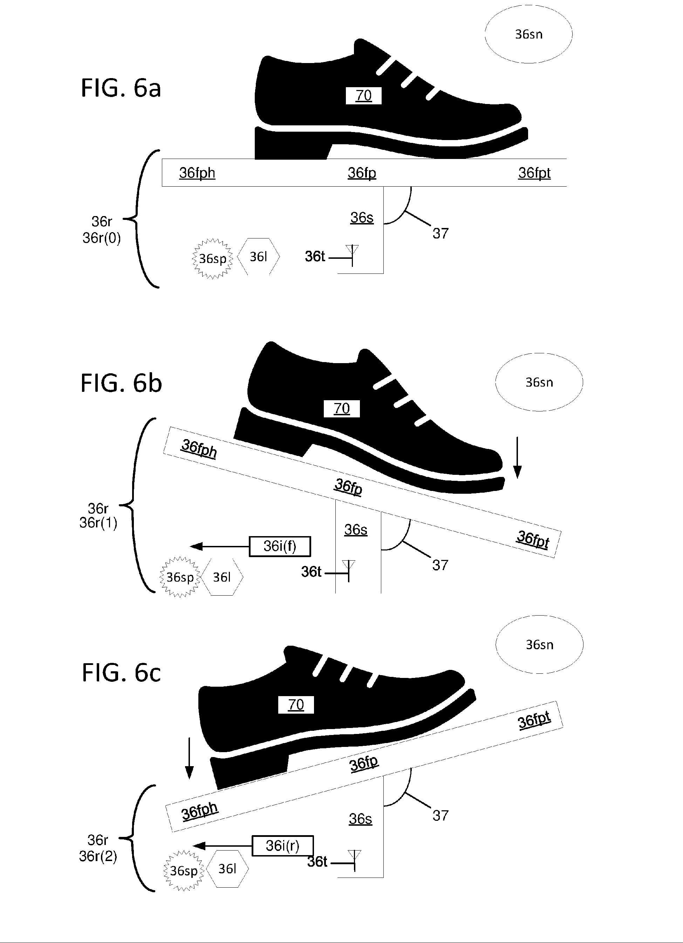

FIGS. 6a-6c are side views of various foot pedal activation positions of a rocker foot pedal 36r. FIG.6a is a side view of rocker foot pedal 36r in a neutral position 36r(0). Rocker foot pedal 36r may include a foot plate 36fp with a toe side 36fpt and a heel side 36fph and a support 36s. Foot plate 36fp may be pivotally attached to and/or be supported by support 36s. Foot plate 36fp may be configured to be supported by support 36s in a neutral position 36r(0), perpendicular to support 36s with an angle 37 between support 36s and toe side 36fpt of foot plate 36fp of about 90 degrees, when no force is applied to foot plate 36fp by a foot 70 of a user. Rocker foot pedal 36r may not generate an input signal when foot plate 36fp is not moved and is in the neutral position 36r(0).

FIG.6b is a side view of rocker foot pedal 36r in a forward activation position. Foot plate 36fp may be configured to pivot and move to a forward activation position 36r(1) when a force is applied to toe side 36fpt of foot plate 36fp by a foot 70 of a user. Rocker foot pedal 36r may generate a noise by a speaker 36sp and/or illuminate a light 361 when foot plate 36fp is moved to forward activation position 36r(1). Forward activation position 36r(1) may be associated with a specific movement or instrument function of robotic arms 40 of FIG. 1. An angle 37 between support 36s and a toe side 36fpt of foot plate 36fp may be less than about 90 degrees. Rocker foot pedal 36r may generate a forward input signal 36i(f) when foot plate 36fp is moved to forward activation position 36r(1). Rocker foot pedal 36r may send forward input signal 36i(f) to surgical console 30, control tower 20 and/or at least one of the robotic arms 40 of FIG. 1 by transmitter 36t of rocker foot pedal 36r. Forward input signal 36i(f) from rocker foot pedal 36r may be utilized by surgical console 30 to remotely control a specific movement or instrument function of robotic arms 40 of FIG. 1. Instrument function, remotely controlled by forward input signal 36i(f) from rocker foot pedal 36r, may include bipolar coagulation, tissue cutting, stapling, monopolar power level, ultrasonic power level, etc.

Once again, while a wireless transmitter 36t is illustrated, it is contemplated and within the scope of the disclosure that hardwired connections may be used to send forward input signal 36i(f) to the surgical console 30 the control tower 20 and/or the robotic arm(s) 40.

FIG.6c is a side view of rocker foot pedal 36r in a reverse activation position. Foot plate 36fp may be configured to pivot and move to a reverse activation position 36r(2) when a force is applied to a heel side 36fph of foot plate 36fp by a foot 70 of a user. Rocker foot pedal 36r may generate a noise by speaker 36sp and/or illuminate light 361 when foot plate 36fp is moved to reverse activation position 36r(2). A noise and/or illumination generated by rocker foot pedal 36r may be different for forward activation position 36r(1) than for reverse activation position 36r(2) so that a user can easily distinguish between the two activation positions. For example, rocker foot pedal 36r may generate a click noise by speaker 36sp when foot plate 36fp is moved to forward activation position 36r(1) and rocker foot pedal 36r may generate a buzzer noise by speaker 36sp when foot plate 36fp is moved to reverse activation position 36r(2). Reverse activation position 36r(2) may be associated with a specific movement or instrument function of robotic arms 40 of FIG. 1. An angle 37 between support 36s and toe side 36fpt of foot plate 36fp may be greater than about 60 degrees. Rocker foot pedal 36r may generate a reverse input signal 36i(r) when foot plate 36fp is moved to reverse activation position 36r(2). Rocker foot pedal 36r may send reverse input signal 36i(r) to surgical console 30, control tower 20 and/or at least one of the robotic arms 40 of FIG. 1 by transmitter 36t of rocker foot pedal 36r. As previously stated, hardwired connections may be used to send reverse input signal 36i(r) to the surgical console 30 the control tower 20 and/or the robotic arm(s) 40. Reverse input signal 36i(r) from rocker foot pedal 36r may be utilized by surgical console 30 to remotely control a specific movement or instrument function of robotic arms 40 of FIG. 1, different from the movement or function controlled by forward input signal 36i(f). Instrument function remotely controlled by reverse input signal 36i(r) from rocker foot pedal 36r may include bipolar coagulation, tissue cutting, stapling, monopolar power level, ultrasonic power level, etc.

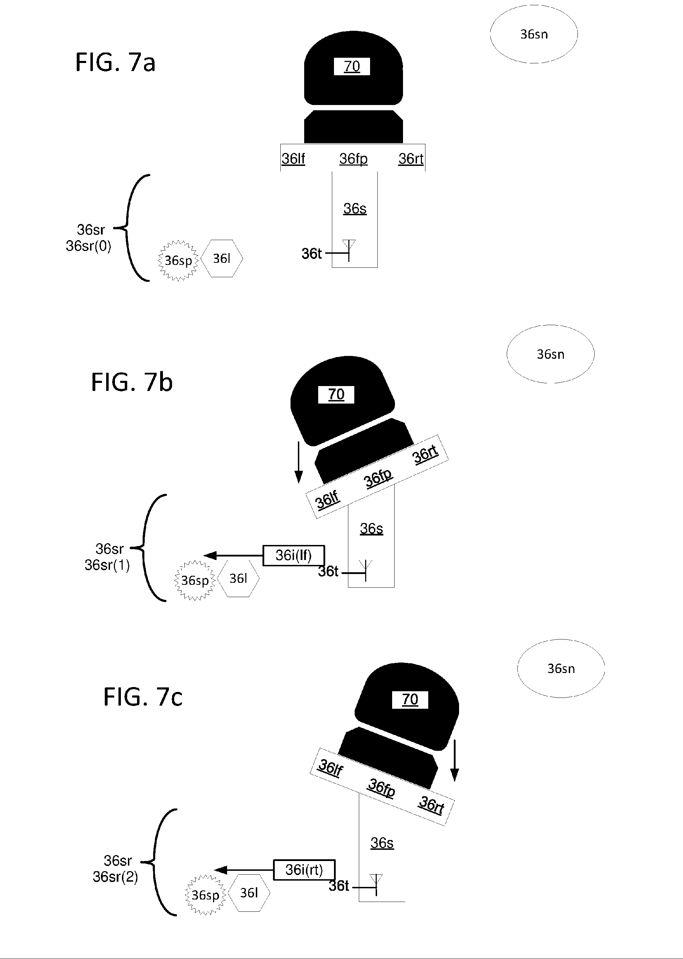

FIGS. 7a-7c are rear views of various foot pedal activation positions of a side rocker foot pedal 36sr. FIG.7a is a rear view of side rocker foot pedal 36sr in a neutral position 36sr(0). Side rocker foot pedal 36sr may include a foot plate 36fp with a left side 361f and a right side 36rt and a support 36s. Foot plate 36fp may be pivotally attached to and/or be supported by support 36s. Foot plate 36fp may be configured to be supported by support 36s in a neutral position 36r(0), perpendicular to support 36s, when no force is applied to foot plate 36fp by a foot 70 of a user. Side rocker foot pedal 36r may not generate an input signal when foot plate 36fp is not moved and is in the neutral position 36sr(0).

FIG.7b is a rear view of side rocker foot pedal 36sr in a left activation position. Foot plate 36fp may be configured to pivot and move to a left activation position 36r(lf) when a force is applied to left side 361f of foot plate 36fp by a foot 70 of a user. Side rocker foot pedal 36sr may generate a noise by speaker 36sp and/or illuminate light 361 when foot plate 36fp is moved to left activation position 36sr(1). Left activation position 36sr(1) may be associated with a specific movement or instrument function of robotic arms 40 of FIG. 1. Side rocker foot pedal 36sr may generate a left input signal 36i(lf) when foot plate 36fp is moved to left activation position 36sr(1). Side rocker foot pedal 36sr may send left input signal 36i(f) to surgical console 30, control tower 20 and/or at least one of the robotic arms 40 of FIG. 1 by transmitter 36t of side rocker foot pedal 36sr. Left input signal 36i(lf) from side rocker foot pedal 36sr may be utilized by surgical console 30 to remotely control a specific movement or instrument function of robotic arms 40 of FIG. 1. Instrument function, remotely controlled by left input signal 36i(lf) from side rocker foot pedal 36sr, may include bipolar coagulation, tissue cutting, stapling, monopolar power level, ultrasonic power level, etc., or combinations thereof.

Once again, while a wireless transmitter 36t is illustrated, it is contemplated and within the scope of the disclosure that hardwired connections may be used to send left input signal 36i(lf) to the surgical console 30 the control tower 20 and/or the robotic arm(s) 40.

FIG.7c is a rear view of side rocker foot pedal 36sr in a right activation position. Foot plate 36fp may be configured to pivot and move to a right activation position 36sr(2) when a force is applied to right side 36rt of foot plate 36fp by a foot 70 of a user. Side rocker foot pedal 36sr may generate a noise by speaker 36sp and/or illuminate light 361 when foot plate 36fp is moved to right activation position 36sr(2). A noise and/or illumination generated by side rocker foot pedal 36sr may be different for left activation position 36sr(1) than for right activation position 36sr(2) so that a user can easily distinguish between the two activation positions. For example, side rocker foot pedal 36sr may generate a click noise by speaker 36sp when foot plate 36fp is moved to left activation position 36sr(1) and side rocker foot pedal 36sr may generate a buzzer noise by speaker 36sp when foot plate 36fp is moved to right activation position 36sr(2). Right activation position 36sr(2) may be associated with a specific movement or instrument function of robotic arms 40 of FIG. 1. Side rocker foot pedal 36sr may generate a right input signal 36i(rt) when foot plate 36fp is moved to right activation position 36sr(2). Side rocker foot pedal 36sr may send right input signal 36i(rt) to surgical console 30, control tower 20 and/or at least one of the robotic arms 40 of FIG. 1 by transmitter 36t of rocker foot pedal 36r. As previously stated, hardwired connections may be used to send right input signal 36i(rt) to the surgical console 30 the control tower 20 and/or the robotic arm(s) 40. Right input signal 36i(rt) from side rocker foot pedal 36sr may be utilized by surgical console 30 to remotely control a specific movement or instrument function of robotic arms 40 of FIG. 1, different from the movement or function controlled by left input signal 36i(lf). Instrument function remotely controlled by right input signal 36i(rt) from side rocker foot pedal 36sr may include bipolar coagulation, tissue cutting, stapling, monopolar power level, ultrasonic power level, etc., or combinations thereof.

In an embodiment, rocker foot pedal 36r shown in FIGS. 6a-6c may be configured to also operate as side rocker foot pedal 36sr shown in FIGS. 7a-7c. Rocker foot pedal 36r may include a selector to select a rocker function of front to back or side to side.

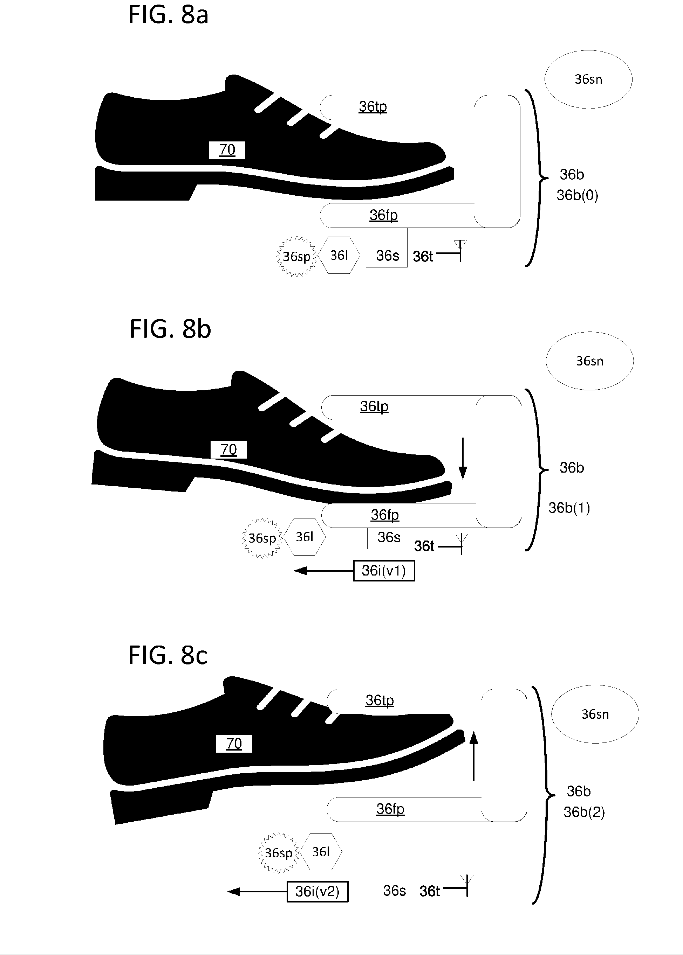

FIGS. 8a-8c are side views of various foot pedal activation positions of a two-stage foot pedal 36b. FIG.8a is a side view of two-stage foot pedal 36b in a neutral position 36b(0). Two-stage foot pedal 36b may include foot plate 36fp attached to a top plate 36tp and a support 36s. Foot plate 36fp may be attached to and/or be supported by support 36s. Foot plate 36fp may be configured to be supported by support 36s in a neutral position 36r(0), perpendicular to support 36s, when no force is applied to foot plate 36fp or top plat 36tp by a foot 70 of a user. Two-stage foot pedal 36b may not generate an input signal when foot plate 36fp or top plate 36tp are not moved and are in neutral position 36b(0).

FIG.8b is a side view of two-stage foot pedal 36b in a down activation position. Foot plate 36fp may be configured to move to a first activation position 36b(1) when a force is applied to move foot plate 36fp and top plate 36tp to first activation position 36b(1) by a foot 70 of a user. Two-stage foot pedal 36b may generate a noise by speaker 36sp and/or illuminate light 361 when foot plate 36fp and top plate 36tp are moved to first activation position 36b(1). First activation position 36b(1) may be associated with a specific movement or instrument function of robotic arms 40 of FIG. 1. Two-stage foot pedal 36b may generate a down input signal 36i(d) when foot plate 36fp and top plate 36tp are moved to first activation position 36b(1). Two-stage foot pedal 36b may send down input signal 36i(d) to surgical console 30, control tower 20 and/or at least one of the robotic arms 40 of FIG. 1 by transmitter 36t of two-stage foot pedal 36b. Down input signal 36i(d) from two-stage foot pedal 36b may be utilized by surgical console 30 to remotely control a specific movement or instrument function of robotic arms 40 of FIG. 1. Instrument function, remotely controlled by down input signal 36i(d) from two-stage foot pedal 36b, may include bipolar coagulation, tissue cutting, stapling, monopolar power level, ultrasonic power level, etc., or combinations thereof.

Once again, while a wireless transmitter 36t is illustrated, it is contemplated and within the scope of the disclosure that hardwired connections may be used to send down input signal 36i(d) to the surgical console 30 the control tower 20 and/or the robotic arm(s) 40.

FIG.8c is a side view of two-stage foot pedal 36b in a second activation position. Foot plate 36fp and top plate 36tp may be configured to move to a second activation position 36b(2) when a force is applied to move top plate 36tp and foot plate 36fp to second activation position 36b(2) by a foot 70 of a user. Two-stage foot pedal 36b may generate a noise by speaker 36sp and/or illuminate light 361 when foot plate 36fp and top plate 36tp are moved to second activation position 36b(2). A noise and/or illumination generated by two-stage foot pedal 36b may be different for first activation position 36b(1) than for second activation position 36b(2) so that a user can easily distinguish between the two activation positions. For example, two-stage foot pedal 36b may generate a click noise by speaker 36sp when foot plate 36fp and top plate 36tp are moved to first activation position 36b(1) and two-stage foot pedal 36b may generate a buzzer noise by speaker 36sp when foot plate 36fp and top plate 36tp are moved to second activation position 36b(2). Second activation position 36b(2) may be associated with a specific movement or instrument function of robotic arms 40 of FIG. 1. Two-stage foot pedal 36b may generate an up input signal 36i(u) when foot plate 36fp and top plate 36tp are moved to second activation position 36b(2). Two-stage foot pedal 36b may send up input signal 36i(u) to surgical console 30, control tower 20 and/or at least one of the robotic arms 40 of FIG. 1 by transmitter 36t of two-stage foot pedal 36b. As previously stated, hardwired connections may be used to send up input signal 36i(u) to the surgical console 30 the control tower 20 and/or the robotic arm(s) 40. Up input signal 36i(u) from two-stage foot pedal 36b may be utilized by surgical console 30 to remotely control a specific movement or instrument function of robotic arms 40 of FIG. 1, different from the movement or function controlled by down input signal 36i(d). Instrument function remotely controlled by up input signal 36i(u) from two-stage foot pedal 36b may include bipolar coagulation, tissue cutting, stapling, monopolar power level, ultrasonic power level, etc., or combinations thereof.

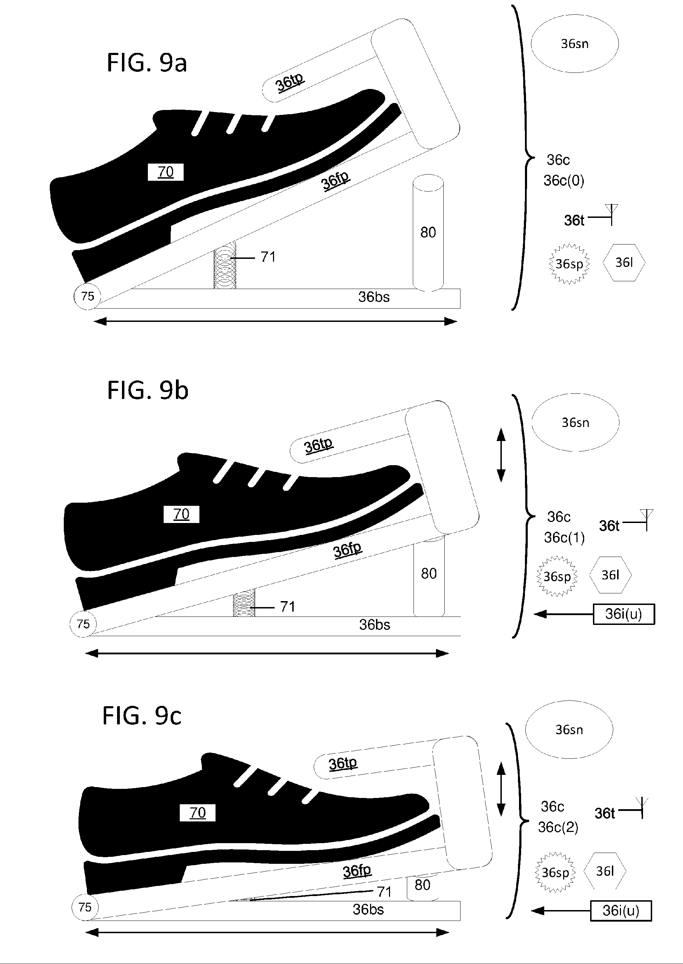

FIGS. 9a-9c are side views of various foot pedal activation positions of a variable output foot pedal 36c. FIG.9a is a side view of variable output foot pedal 36c in a neutral position 36c(0). Variable output foot pedal 36c may include foot plate 36fp attached to top plate 36tp, a base 36bs, a spring, 70, a hinge 75 and a switch 80. Foot plate 36fp may be attached to base 36bs by hinge 75 and spring 71. Foot plate 36fp may be configured to be supported by hinge 75 and spring 71 in a neutral position 36c(0)when no force is applied to foot plate 36fp or top plat 36tp by a foot 70 of a user. Variable output foot pedal 36c may not generate an input signal when foot plate 36fp or top plate 36tp are not moved and are in neutral position 36c(0).

FIG.9b is a side view of variable foot pedal 36c in a first activation position. Foot plate 36fp may be configured to move to a first activation position 36b(1) of switch 80 when a force is applied to move foot plate 36fp and top plate 36tp to first activation position 36c(1) by a foot 70 of a user. Variable output foot pedal 36c may generate a noise by speaker 36sp and/or illuminate light 361 when foot plate 36fp and top plate 36tp are moved to first activation position 36c(1). First activation position 36c(1) may be associated with a specific movement or instrument function of robotic arms 40 of FIG. 1. Variable output foot pedal 36c may generate a first input signal 36i(vl) when foot plate 36fp and top plate 36tp are moved to first activation position 36v(1). Variable output foot pedal 36c may send first input signal 36i(v1) to surgical console 30, control tower 20 and/or at least one of the robotic arms 40 of FIG. 1 by transmitter 36t of Variable output foot pedal 36c. First input signal 36i(v1) from Variable output foot pedal 36c may be utilized by surgical console 30 to remotely control a specific movement or instrument function of robotic arms 40 of FIG. 1. Instrument function, remotely controlled by first input signal 36i(vl) from Variable output foot pedal 36c, may include bipolar coagulation, tissue cutting, stapling, monopolar power level, ultrasonic power level, etc., or combinations thereof.

Once again, while a wireless transmitter 36t is illustrated, it is contemplated and within the scope of the disclosure that hardwired connections may be used to send first input signal 36i(v1) to the surgical console 30 the control tower 20 and/or the robotic arm(s) 40.

FIG.9c is a side view of variable output foot pedal 36c in a second activation position. Foot plate 36fp and top plate 36tp may be configured to move to a second activation position 36c(2) of switch 80 when a force is applied to move top plate 36tp and foot plate 36fp to second activation position 36c(2) by a foot 70 of a user. Variable output foot pedal 36c may generate a noise by speaker 36sp and/or illuminate light 361 when foot plate 36fp and top plate 36tp are moved to second activation position 36c(2). A noise and/or illumination generated by variable output foot pedal 36c may be different for first activation position 36c(1) than for second activation position 36c(2) so that a user can easily distinguish between the two activation positions. Second activation position 36c(2) may be associated with a specific movement or instrument function of robotic arms 40 of FIG. 1. Variable output foot pedal 36c may generate a second input signal 36i(v2) when foot plate 36fp and top plate 36tp are moved to second activation position 36c(2). Variable output foot pedal 36c may send second input signal 36i(v2) to surgical console 30, control tower 20 and/or at least one of the robotic arms 40 of FIG. 1 by transmitter 36t of variable output foot pedal 36c. As previously stated, hardwired connections may be used to send second input signal 36i(v2) to the surgical console 30 the control tower 20 and/or the robotic arm(s) 40. Second input signal 36i(v2) from variable output foot pedal 36c may be utilized by surgical console 30 to remotely control a specific movement or instrument function of robotic arms 40 of FIG. 1, different from the movement or function controlled by first input signal 36i(v1). Instrument function remotely controlled by second input signal 36i(v2) from variable output foot pedal 36c may include bipolar coagulation, tissue cutting, stapling, monopolar power level, ultrasonic power level, etc., or combinations thereof.

While variable output foot pedal 36c is illustrated as moving in a downward direction from neutral position 36c(0) to first activation position 36c(1) and from first activation position 36c(1) to second activation position 36c(2), it is contemplated and within the scope of the disclosure that foot plate 36fp of variable output foot pedal 36c may move in an upward direction from second activation position 36c(2) to first activation position 36c(1) and from first activation position 36c(1) to neutral position 36c(0). Spring 71 and top plate 36tp may assist foot 70 of user to move foot plate 36fp in an upward direction.

It is further contemplated that variable output foot pedal 36c may include additional activation positions to the first and second activation positions illustrated. Further, it is contemplated that foot pedal 36fp of variable output foot pedal 36c may move from neutral position 36c(0) to first activation position 36c(1) and or second activation position 36c(2) by sliding axially along base 36bs rather than pivoting at hinge 75.

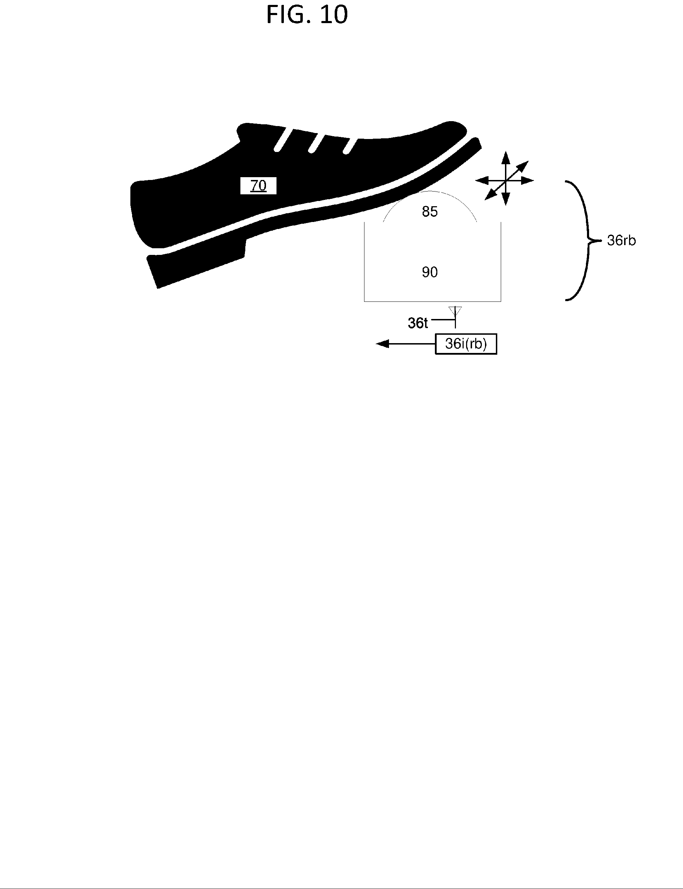

FIG.10 is a side view of a roller ball foot pedal 36rb of the surgical robotic system of FIG. 1 according to an embodiment of the present disclosure. Roller ball foot pedal 36rb may include a roller ball 85 and a housing 90. Roller ball 85 may be configured to rotate within housing 90. A foot 70 of a user may be placed in contact with roller ball 85 and may rotate roller ball 85 within housing 90 in any direction to control a selection of an option displayed on first display 32 or second display 34. Roller ball foot pedal 36rb may send an input 36i(rb) to surgical console 30 or control tower 20 by transmitter 36t to select an option displayed on first display 32 or second display 34. Input 36i(rb) from roller ball foot pedal 36rb may be utilized to select an option such as monopolar power level, ultrasonic power level, etc.

Two-stage foot pedal 36a or 36b, rocker foot pedal 36r, side rocker foot pedal 36sr or variable output foot pedal 36c may be color coded, include an image or other identifier so as to identify a specific movement or instrument function of robotic arms 40 of FIG. 1 assigned to the foot pedal to user of surgical robotic system 10. A color and feature assigned to two-stage foot pedal 36a or 36b, rocker foot pedal 36r, side rocker foot pedal 36sr, or variable output foot pedal 36c may stay with the function of the pedal. In addition, an overlay in a video feed a user sees when operating the surgical robotic system 10, such as those shown on first display 32 or second display 34, may include a display of each of two-stage foot pedal 36a or 36b, rocker foot pedal 36r, side rocker foot pedal 36sr, or variable output foot pedal 36c of surgical robotic system 10 with their locations and color to help the user to locate them. Two-stage foot pedal 36a or 36b, rocker foot pedal 36r, side rocker foot pedal 36sr, or variable output foot pedal 36c may also include a location sensor 36sn at the pedal location to indicate a location of a foot 70 of a user relative to Two-stage foot pedal 36a or 36b, rocker foot pedal 36r, side rocker foot pedal 36sr, or variable output foot pedal 36c in real time and ensure the foot 70 is stepping on the correct pedal. Video display of first display 32 or second display 34 may also provide a visual indication and change to indicate when two-stage foot pedal 36a or 36b, rocker foot pedal 36r, side rocker foot pedal 36sr, or variable output foot pedal 36c is depressed. Location sensor 36sn may be a capacitive proximity sensor in two-stage foot pedal 36a or rocker foot pedal 36r and may be less susceptible to small dirt and debris. Location sensor 36sn may be an optical sensor with reflected light or a video camera system looking at a user's feet 70 and include software for determining foot 70 location.

In some embodiments, two-stage foot pedal 36a or 36b, rocker foot pedal 36r, side rocker foot pedal 36sr, or variable output foot pedal 36c may be configured to illuminate light 361 when a foot 70 hovers over each specific pedal. Light 361 may flash when two-stage foot pedal 36a or rocker foot pedal 36r is depressed and activated. Light 361 may flash at different speeds to indicate a power level or function associated with two-stage foot pedal 36a or rocker foot pedal 36r and a foot pedal image may flash when displayed on first display 32 or second display 34.

In another embodiment, a user may utilize a force driven joystick or stick-type controller (not shown) with first display 32 or second display 34 and an input from two-stage foot pedal 36a or 36b, rocker foot pedal 36r, side rocker foot pedal 36sr, or variable output foot pedal 36c may be utilized to select a force input or power level.

A device in accordance with the present disclosure may provide a user with the ability to control multiple movements or instrument functions of the robotic arms 40 of a robotic surgical system 10 with a foot pedal. A device in accordance with the present disclosure may provide a user with the ability to control different movements or instrument functions of the robotic arm 40 of a robotic surgical system by depressing a foot pedal to either a first activation position or a second activation position. A device in accordance with the present disclosure may provide a user with the ability to control different movements or instrument functions of the robotic arm of a robotic surgical system by depressing a foot pedal to either forwards or backwards. A device in accordance with the present disclosure may provide a user with a visual indication of when a user's foot is hovering over a foot pedal to determine that the correct foot pedal is being used. A device in accordance with the present disclosure may provide a user with indicator when a foot pedal is depressed.

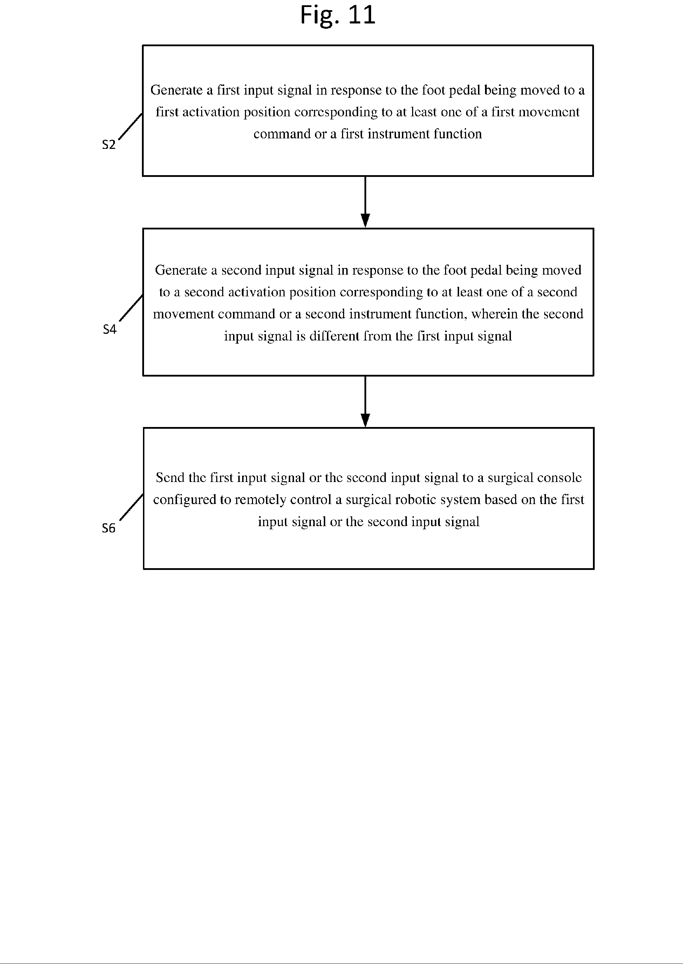

FIG.11 illustrates a flow diagram of a method for controlling a movement or instrument function of a robotic arm 40 of a robotic surgical system 10, arranged in accordance with at least some embodiments presented herein. The method may include one or more operations, actions, or functions as illustrated by one or more of blocks S2, S4, and/or S6. Although illustrated as discrete blocks, various blocks may be divided into additional blocks, combined into fewer blocks, or eliminated, depending on the desired implementation.

Processing may begin at block S2, "Generate a first input signal in response to the foot pedal being moved to a first activation position corresponding to at least one of a first movement command or a first instrument function." At block S2, a foot pedal may generate a first input signal in response to the foot pedal (e.g., two-stage foot pedal 36a or rocker foot pedal 36r) being moved by a foot of a user to a first activation position. The first activation position may be associated with a first specific movement or instrument function of the surgical robotic system 10. The foot pedal may be a two-stage foot pedal 36a, a rocker foot pedal 36r, or a foot pedal configured to control more than one function.

Processing may continue from block S2 to block S4, "Generate a second input signal in response to the foot pedal being moved to a second activation position corresponding to at least one of a second movement command or a second instrument function, wherein the second input signal is different from the first input signal." At block S4, the foot pedal 36a/36r may generate a second input signal in response to the foot pedal 36a/36r being moved by the foot of the user to a second activation position. The second activation position may be associated with a second specific movement or instrument function of the surgical robotic system 10. The second input signal may be different from the first input signal.

Processing may continue from block S4 to block S6, "Send the first input signal or the second input signal to a surgical console configured to remotely control a surgical robotic system based on the first input signal or the second input signal." At block S6, the foot pedal 36a/36r may send the first input signal or the second input signal to a surgical console of the surgical robotic system 10. The surgical console may utilize the first input signal or the second input signal to remotely control the respective specific movement or instrument function of the robotic arm 40.

It will be understood that various modifications may be made to the embodiments disclosed herein. In embodiments, the sensors may be disposed on any suitable portion of the robotic arm. Therefore, the above description should not be construed as limiting, but merely as exemplifications of various embodiments. Those skilled in the art will envision other modifications within the scope and spirit of the claims appended thereto.

The invention may be described by reference to the following numbered paragraphs:-
1. A foot pedal system for a surgical robotic system, the foot pedal system comprising:
   a foot pedal with a foot plate and a support, wherein the foot plate includes a first portion and a second portion, and the foot pedal is configured to:
   generate a first input signal in response to the foot pedal being moved to a first activation position corresponding to at least one of a first movement command or a first instrument function;
   generate a second input signal in response to the foot pedal being moved to a second activation position corresponding to at least one of a second movement command or a second instrument function, wherein the second input signal is different from the first input signal; and
   send the first input signal or the second input signal to a surgical console configured to remotely control a surgical robotic system based on the first input signal or the second input signal.
2. The foot pedal system of paragraph 1, wherein the foot pedal is a two-stage foot pedal.
3. The foot pedal system of paragraph 2, wherein the two-stage foot pedal is configured to move to the first activation position and generate a physical click when a force is applied to move the foot plate to the first activation position by a foot of a user and to move to the second activation position and generate a physical click when a force is applied to move the foot plate to the second activation position by the foot of the user.
4. The foot pedal system of paragraph 1, wherein the foot pedal is a rocker foot pedal.
5. The foot pedal system of paragraph 4, wherein the rocker foot pedal is configured to pivot to the first activation position when a force is applied to move the first portion of the foot plate to the first activation position by a foot of a user and to pivot to the second activation position when a force is applied to the second portion of the foot plate to move the second portion of the foot plate to the second activation position by the foot of the user.
6. The foot pedal system of paragraph 1, wherein the surgical robotic system includes a robotic arm and the foot pedal is color coded to identify specific movements or instrument functions of the robotic arm assigned to the foot pedal.
7. The foot pedal system of paragraph 1, further comprising a location sensor to indicate a location of a foot relative to the foot pedal.
8. The foot pedal system of paragraph 7, wherein the location sensor is one of a capacitive proximity sensor, an optical sensor with reflected light, or a video camera system.
9. The foot pedal system of paragraph 8 wherein the foot pedal further comprises a light configured to illuminate in response to at least one of a foot hovering over the foot pedal or the foot pedal being moved to the first activation position or the second activation position.
10. The foot pedal system of paragraph 1, wherein the instrument function includes at least one of bipolar coagulation, tissue cutting, stapling, monopolar power level, or ultrasonic power level.
11. A surgical robotic system, the system comprising:
   a robotic arm including a surgical instrument; and
   a surgical console including at least one foot pedal;
      wherein the foot pedal is configured to:
         generate a first input signal in response to the foot pedal being moved to a first activation position corresponding at least one of a first movement command or a first instrument function;
         generate a second input signal in response to the foot pedal being moved to a second activation position corresponding to at least one of a second movement command or a second instrument function, wherein the second input signal is different from the first input signal;
         send the first input signal or the second input signal to a surgical console configured to remotely control a surgical robotic system based on the first input signal or the second input signal; and
      a controller configured to:
         control the robotic arm based on at least one of the first movement command or the second movement command; and
         control the instrument based on at least one of the first instrument function or the second instrument function.
12. The system of paragraph 11, wherein the foot pedal is a two-stage foot pedal and is configured to move to the first activation position and generate a physical click when a force is applied to move a foot plate of the foot pedal to the first activation position by a foot of a user and to move to the second activation position and generate a physical click when a force is applied to move the foot plate to the second activation position by the foot of the user.
13. The system of paragraph 11, wherein the foot pedal is a rocker foot pedal and is configured to pivot to a first activation position when a force is applied to move a toe side of a foot plate to the first activation position by a foot of a user and to pivot to the second activation position when a force is applied to move a heel side of the foot plate to the second activation position by the foot of the user.
14. The system of paragraph 11, wherein the foot pedal is color coded to identify specific movements or instrument functions of the robotic arm assigned to the foot pedal.
15. The system of paragraph 14, wherein a video display of the surgical robotic system displays the foot pedal and the color code of the foot pedal.
16. The system of paragraph 11, wherein the foot pedal further comprises a location sensor to indicate a location of a foot relative to the foot pedal.
17. The system of paragraph 16, wherein the location sensor is one of a capacitive proximity sensor, an optical sensor with reflected light, or a video camera system.
18. The system of paragraph 11, wherein the instrument function includes at least one of bipolar coagulation, tissue cutting, stapling, monopolar power level, or ultrasonic power level.
19. A method for controlling a movement or instrument function of a robotic arm of a surgical robotic system with a foot pedal, the method comprising:
   generating a first input signal in response to the foot pedal being moved to a first activation position corresponding to at least one of a first movement command or a first instrument function;
   generating a second input signal in response to the foot pedal being moved to a second activation position corresponding to at least one of a second movement command or a second instrument function, wherein the second input signal is different from the first input signal; and
   sending the first input signal or the second input signal to a surgical console configured to remotely control a surgical robotic system based on the first input signal or the second input signal.
20. The method of paragraph 19 wherein the instrument function includes at least one of bipolar coagulation, tissue cutting, stapling, monopolar power level, or ultrasonic power level.

## Claims

1. A foot pedal system for a surgical robotic system, the foot pedal system comprising:
a foot pedal with a foot plate and a support, wherein the foot plate includes a first portion and a second portion, and the foot pedal is configured to:
generate a first input signal in response to the foot pedal being moved to a first activation position corresponding to at least one of a first movement command or a first instrument function;
generate a second input signal in response to the foot pedal being moved to a second activation position corresponding to at least one of a second movement command or a second instrument function, wherein the second input signal is different from the first input signal; and
send the first input signal or the second input signal to a surgical console configured to remotely control a surgical robotic system based on the first input signal or the second input signal.

2. The foot pedal system of claim 1, wherein the foot pedal is a two-stage foot pedal.

3. The foot pedal system of claim 2, wherein the two-stage foot pedal is configured to move to the first activation position and generate a physical click when a force is applied to move the foot plate to the first activation position by a foot of a user and to move to the second activation position and generate a physical click when a force is applied to move the foot plate to the second activation position by the foot of the user.

4. The foot pedal system of any preceding claim, wherein the foot pedal is a rocker foot pedal; preferably wherein the rocker foot pedal is configured to pivot to the first activation position when a force is applied to move the first portion of the foot plate to the first activation position by a foot of a user and to pivot to the second activation position when a force is applied to the second portion of the foot plate to move the second portion of the foot plate to the second activation position by the foot of the user.

5. The foot pedal system of any preceding claim, wherein the surgical robotic system includes a robotic arm and the foot pedal is color coded to identify specific movements or instrument functions of the robotic arm assigned to the foot pedal.

6. The foot pedal system of any preceding claim, further comprising a location sensor to indicate a location of a foot relative to the foot pedal; preferably wherein the location sensor is one of a capacitive proximity sensor, an optical sensor with reflected light, or a video camera system.

7. The foot pedal system of claim 6 wherein the foot pedal further comprises a light configured to illuminate in response to at least one of a foot hovering over the foot pedal or the foot pedal being moved to the first activation position or the second activation position.

8. The foot pedal system of any preceding claim, wherein the instrument function includes at least one of bipolar coagulation, tissue cutting, stapling, monopolar power level, or ultrasonic power level.

9. A surgical robotic system, the system comprising:
a robotic arm including a surgical instrument; and
a surgical console including at least one foot pedal;
wherein the foot pedal is configured to:
generate a first input signal in response to the foot pedal being moved to a first activation position corresponding at least one of a first movement command or a first instrument function;
generate a second input signal in response to the foot pedal being moved to a second activation position corresponding to at least one of a second movement command or a second instrument function, wherein the second input signal is different from the first input signal;
send the first input signal or the second input signal to a surgical console configured to remotely control a surgical robotic system based on the first input signal or the second input signal; and
a controller configured to:
control the robotic arm based on at least one of the first movement command or the second movement command; and
control the instrument based on at least one of the first instrument function or the second instrument function.

10. The system of claim 9, wherein the foot pedal is a two-stage foot pedal and is configured to move to the first activation position and generate a physical click when a force is applied to move a foot plate of the foot pedal to the first activation position by a foot of a user and to move to the second activation position and generate a physical click when a force is applied to move the foot plate to the second activation position by the foot of the user; preferably wherein the foot pedal is a rocker foot pedal and is configured to pivot to a first activation position when a force is applied to move a toe side of a foot plate to the first activation position by a foot of a user and to pivot to the second activation position when a force is applied to move a heel side of the foot plate to the second activation position by the foot of the user.

11. The system of claim 9 or claim 10, wherein the foot pedal is color coded to identify specific movements or instrument functions of the robotic arm assigned to the foot pedal; preferably wherein a video display of the surgical robotic system displays the foot pedal and the color code of the foot pedal.

12. The system of any of claims 9 to 11, wherein the foot pedal further comprises a location sensor to indicate a location of a foot relative to the foot pedal; preferably wherein the location sensor is one of a capacitive proximity sensor, an optical sensor with reflected light, or a video camera system.

13. The system of any of claims 9 to 12, wherein the instrument function includes at least one of bipolar coagulation, tissue cutting, stapling, monopolar power level, or ultrasonic power level.

14. A method for controlling a movement or instrument function of a robotic arm of a surgical robotic system with a foot pedal, the method comprising:
generating a first input signal in response to the foot pedal being moved to a first activation position corresponding to at least one of a first movement command or a first instrument function;
generating a second input signal in response to the foot pedal being moved to a second activation position corresponding to at least one of a second movement command or a second instrument function, wherein the second input signal is different from the first input signal; and
sending the first input signal or the second input signal to a surgical console configured to remotely control a surgical robotic system based on the first input signal or the second input signal.

15. The method of claim 14 wherein the instrument function includes at least one of bipolar coagulation, tissue cutting, stapling, monopolar power level, or ultrasonic power level.
